# EUROPEAN PATENT APPLICATION

(11) **EP 1 977 750 A1**
(43) Date of publication of application: **08.10.2008**
(21) Application number: 06841750.0
(22) Date of filing: 19.12.2006
(51) Int. Cl.: A61K 31/426

(54) **USE OF THIOPROLINE IN BODY WEIGHT REDUCTION TREATMENTS**

(30) Priority: 02.01.2006 ES 200600008
(71) Applicant: Universidad de Cadiz, 11001 Cadiz (ES)
(72) Inventor: NAVARRO ARÉVALO, Ana, 11003, Cádiz (ES); BOVERIS, Alberto, Universidad de Buenos Aires (AR)
(74) Representative: ABG Patentes, S.L.
(86) International application number: PCT/ES2006/000698
(87) International publication number: WO 2007/077276

(57) **Abstract**

The invention relates to thioproline, pharmaceutically acceptable salts and/or prodrugs thereof that can be used as anorexia-inducing agents in both therapeutic and cosmetic treatments to reduce body weight.

## Description

### Field of the Invention

The invention relates to the use of thioproline, pharmaceutically acceptable salts and/or prodrugs thereof as an anorexic or anorexia-inducing agent in treatments for reducing body weight for both therapeutic and cosmetic purposes.

### Background of the Invention

Excess body weight attracts particular attention in developed countries today. The current cultural guidelines, which lend significant importance to personal esthetics, emphasize overweight as a less than desirable characteristic. When overweight leads to obesity, clinical medicine faces a serious problem since it is related to important pathologies such as diabetes or hypertension.

Calorie restriction, which is defined as the balanced intake of foods in an amount of at least 20% less than the normal intake, is an effective anti-aging treatment, increasing survival and improving the physiological deficiencies associated to senescence processes.

Although the causes of excess weight and obesity are complex, a common characteristic in the diet of people who are overweight is a caloric intake which exceeds caloric consumption. The methodology combining a restricted diet with increased physical activity is useful for maximizing weight loss. Nevertheless, intake and activity patterns having a counterproductive effect on achieving the weight reduction objective are often developed.

Weight reduction programs generally use appetite-suppressant drugs as coadyuvants in treatment. When an anorexic drug is prescribed to a well-motivated subject, increased acceptance and tolerance for a low-calorie diet is often developed. Nevertheless, in many cases the use of an anorexic agent can cause undesirable side effects, or its effect can decrease after a period of time.

Therefore, it would of great interest to have a novel therapy available that is useful for the treatment of excess body weight. Advantageously, said treatment should have low or no side effects.

Now it has surprisingly been found that thioproline, salts and/or prodrugs thereof can be used in body weight reduction treatments involving diets with a low caloric intake without causing undesirable effects.

Thioproline or thiazolidine-4-carboxylic acid is a normal brain metabolite of 5-hydroxytryptamine catabolism. Some nitroso derivatives of 1-thioproline, such as N-nitroso-1-4-thioproline and N-nitroso-1-4-methyl thioproline, have been identified in human urine.

It has been described that thioproline produces a series of biological effects: (a) inhibition of chemical carcinogenesis, interpreted as an inhibition of *in vivo* nitrosylation of N-benzylmethylamine; (b) improvement of the immune response of old mice, postulated as an antioxidant effect; and (c) increase in the mean life span associated to decreased oxygen consumption in *Drosophila melanogaster,* also explained as an antioxidant action. Nothing in the state of the art suggests the novel application of thioproline as an anorexic agent as now found.

### Summary of the Invention

Now, it has surprisingly been found that thioproline, particularly *1*-thioproline, is capable of exerting an anorexic or anorexia-inducing (anorexigenic) effect in mice (Example 1). This novel application of thioproline is based on research carried out on mice in which thioproline was administered, observing that the food intake was spontaneously reduced in the treated animals, thus reducing their weight while at the same time they responded favorably to neurological evaluation tests and registered a prolongation of their period of life (Examples 1-3) .

Therefore, in one aspect the invention relates to the use of thioproline, salts and/or prodrugs thereof as an anorexic or anorexia-inducing (anorexigenic) agent.

In another aspect, the invention relates to the use of thioproline in the preparation of a pharmaceutical composition for reducing body weight. Said pharmaceutical composition can be used in the treatment of any situation which requires reducing an individual's body weight, for example, in overweight or obesity situations.

In another aspect, the invention relates to a method for improving the bodily appearance of an individual comprising administering thioproline, pharmaceutically acceptable salts and/or prodrugs thereof to said subject in an effective amount for reducing appetite and repeating said dose until achieving a cosmetically beneficial loss of body weight.

The use of thioproline for reducing body weight is an effective means of avoiding the undesirable side effects such as intestinal disorders and restlessness occurring with current treatments.

### Brief Description of the Figures

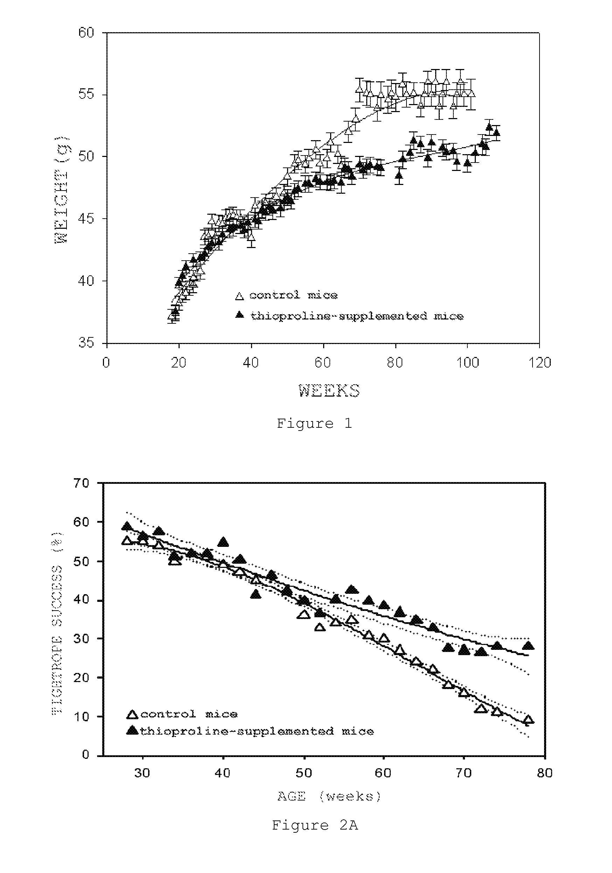

Figure 1 is an illustrative graph of the variation of body weight according to time in mice treated with thioproline. Hyperbola adjustment: control mice, r² = 0.96; thioproline-supplemented mice, r² = 0.97; *t*-test p < 0.05.

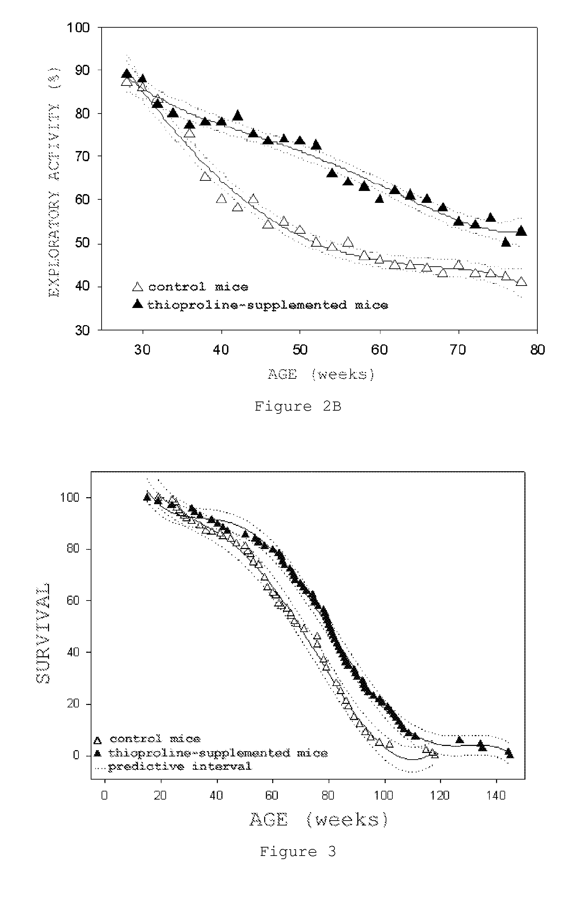

Figure 2 shows the results of the assessment of thioproline on the neurological capacities of thioproline-supplemented mice versus control mice. Figure 2A is a graph showing the effect of supplementing the diet with thioproline on the neuromuscular coordination of mice according to age evaluated by means of the tightrope test: control mice, r² = 0.99; thioproline-supplemented mice, r² = 0.93; t-test p < 0.05. Figure 2B is a graph showing the effect of supplementing the diet with thioproline on the spontaneous cognitive neurological activity of mice according to age evaluated by means of the T maze test: control mice, r² = 0.98; thioproline-supplemented mice, r² = 0.97; t-test p < 0.001.

Figure 3 is an illustrative graph of the survival of thioproline-supplemented mice versus control mice. Control male mice (n = 50): mean life span, 62 ± 4 weeks; maximum life expectancy, 118 ± 4 weeks; thioproline-supplemented mice (n = 70): mean life span, 80 ± 3 weeks; maximum life expectancy, 145 ± 3 weeks. Survival distribution statistics: Log Rank: 9.0, p < 0.001; Breslow: 11.5, p < 0.0001; Tarone-Ware: 11, p < 0.0001.

### Detailed Description of the Invention

In one aspect, the invention relates to the use of thioproline in the preparation of a pharmaceutical composition for reducing body weight. Said pharmaceutical composition can be used for both therapeutic purposes, for example in the treatment of any situation which requires reducing an individual's body weight, for example overweight, obesity, etc., and cosmetic purposes, for example to improve the bodily appearance of a healthy individual who does not need to lose weight but rather must remain at the weight considered normal.

As it is used in this description, the term "individual" relates to a mammal and includes although is not limited to domestic animals, rodents, primates and humans. Preferably, said individual is a human being, male or female, of any age or race.

Thioproline, or thiazolidine-4-carboxylic acid, has a chiral carbon and therefore exists either in the form of its isolated enantiomers (d and 1) or in the form of mixtures of said enantiomers. Said mixtures can be racemic mixtures, such as mixtures enriched with any one of said enantiomers. In a particular embodiment, an enantiomeric mixture (such as a racemic mixture or a mixture enriched with one of the d or *1* enantiomers of thioproline) is used. In another particular embodiment, the *1* enantiomer of thioproline (1-thioproline) is used. Thioproline is a commercially available product that can be supplied by the Sigma-Aldrich Inc company for example.

Likewise, as it is used herein the term "pharmaceutically acceptable salts" relates to any thioproline salt which can be used in the preparation of a pharmaceutical composition. The nature of the salt is not critical provided that it is pharmaceutically acceptable.

Thioproline has an acid group and a base group, so it can react with organic or inorganic bases and with organic or inorganic acids and form the corresponding salts. In a particular embodiment, said salts are pharmaceutically acceptable acid or base addition salts.

The pharmaceutically acceptable acid addition salts can be obtained by reacting thioproline with the suitable acid, by conventional methods well known by persons skilled in the art, in the suitable stoichiometric amount. Illustrative, non-limiting examples of acids that can be used for obtaining said pharmaceutically acceptable acid addition salts include organic acids, for example ascorbic acid, citric acid, methanesulfonic acid, p-toluenesulfonic acid, etc., or inorganic acids, for example hydrochloric acid, hydrobromic acid, sulfuric acid, etc.

The pharmaceutically acceptable base addition salts can be obtained by reacting thioproline with the suitable base, by conventional methods well known by persons skilled in the art, in the suitable stoichiometric amount. Bases useful for the formation of pharmaceutically acceptable base addition salts are known by persons skilled in the art and include amines, etc.

Other pharmaceutically acceptable salts of thioproline are pharmaceutically acceptable metal salts formed by reacting thioproline with an inorganic base in the suitable stoichiometric amount, such as, for example, salts formed with the ions of sodium, potassium, calcium, magnesium, aluminum, etc.

Other pharmaceutically acceptable salts of thioproline are the pharmaceutically acceptable amine salts formed by reacting thioproline with ammonia or with an appropriate nitrogenous base, such as a base strong enough to form a salt with a carboxylic acid, in the suitable stoichiometric amount, such as, for example, salts formed with ammonium, etc.

The prodrugs of thioproline are also within the scope of this invention. As it is used herein, the term "prodrug" includes any compound derived from thioproline, for example, an ester, an amide, etc., which, when administered to an individual, is capable of directly or indirectly providing thioproline, or one of the pharmaceutically acceptable salts thereof, in said individual. Advantageously, said derivative is a compound which increases the bioavailability of thioproline when administered to an individual or which enhances the release of thioproline in a biological compartment. The nature of said derivative is not critical provided that it can be administered to an individual and provides thioproline in a biological compartment of an individual. The preparation of said prodrug can be done by means of conventional methods known by persons skilled in the art. In a particular embodiment, said thioproline prodrug is an ester of thioproline which can be obtained by conventional methods of ester formation known by persons skilled in the art. Non-limiting examples of esters of thioproline useful to put the present invention into practice include pharmaceutically acceptable, low molecular weight alkyl esters, for example, esters of alkyl groups with 5 or less carbon atoms, for example, methyl, ethyl, propyl, etc. In another particular embodiment, said thioproline prodrug is an amide of thioproline which can be obtained by conventional methods of amide formation known by persons skilled in the art. Non-limiting examples of amides of thioproline useful to put the present invention into practice include amides formed with pharmaceutically acceptable, low molecular weight amines, for example, amines with 5 or less carbon atoms, for example, methylamine, ethylamine, propylamine, etc.

So that it can be administered to an individual, thioproline will be in a pharmaceutically acceptable or substantially pure form, i.e. the thioproline will have a pharmaceutically acceptable purity level excluding the pharmaceutically acceptable excipients and not including material considered toxic at the normal dosage levels. The purity levels for thioproline are preferably greater than 50%, more preferably greater than 70%, more preferably greater than 90%, even more preferably greater than 95% of thioproline.

In general, for its administration to an individual for the purpose of reducing the body weight of said individual, for therapeutic or cosmetic purposes, the thioproline, pharmaceutically acceptable salts and/or prodrugs thereof will be formulated in an appropriate pharmaceutical composition, in the effective amount, together with one or more pharmaceutically acceptable carriers, adjuvants or excipients. Examples of said pharmaceutical compositions include solid (e.g. tablets, sugar-coated tablets, capsules, etc.), liquid (e.g. solutions, suspensions, emulsions, etc.) formulations, etc., for its administration by any appropriate administration route, for example by oral route, by parenteral route (e.g. intramuscular, subcutaneous, intravenous, etc.), rectal, etc.

In a particular embodiment, said pharmaceutical compositions can be in an oral dosage form, either in solid or liquid form. Illustrative examples of oral dosage forms include tablets, capsules, granulates, solutions, suspensions, etc., and can contain the conventional excipients, such as binders, diluents, disintegrants, lubricants, wetting agents, etc., and can be prepared by conventional methods. The pharmaceutical compositions can also be adapted for their parenteral administration in the form of sterile solutions, suspensions or lyophilized products, for example, in the appropriate dosage form; in this case, said pharmaceutical compositions will include the suitable excipients, such as buffers, surfactants, etc. In any case, the excipients will be chosen according to the selected pharmaceutical dosage form. A review of the different pharmaceutical dosage forms of drugs and of their preparation can be found in the book "Tratado de Farmacia Galénica", by C. Faulí i Trillo, 10^{th} Edition, 1993, Luzán 5, S.A. de Ediciones.

In general, the effective amount of thioproline, pharmaceutically acceptable salts and/or prodrugs thereof, to be administered will depend on, among other factors, the individual to be treated, on the chosen dosage form, etc. For that reason, the mentioned doses in this invention must be considered only as a guideline for a person skilled in the art, and he must adjust the doses according to the previously mentioned variables. Nevertheless, thioproline can be administered one or more times a day, for example 1 or 2 times a day, in a typical total daily amount comprised between 500 and 1,000 mg/day.

The administration of thioproline, pharmaceutical salts and/or prodrugs thereof, can be done alone or in combination with additional drugs useful for reducing body weight to provide a combined therapy. Said additional drugs can form part of the same pharmaceutical composition comprising thioproline, pharmaceutical salts and/or prodrugs thereof provided by this invention, or they can alternatively be provided in the form of a separate pharmaceutical composition for its simultaneous or sequential administration in relation to the pharmaceutical composition comprising thioproline, pharmaceutical salts and/or prodrugs thereof provided by this invention. Illustrative, non-limiting examples of said additional drugs that can be used to provide combination therapy include anorexia-inducing agents, appetite suppressants, etc.

In another aspect, the invention relates to a method for improving the bodily appearance of an individual comprising administering thioproline, pharmaceutically acceptable salts and/or prodrugs thereof to said individual in an effective amount for reducing appetite and repeating said dose until achieving a cosmetically beneficial loss of body weight.

In this case, the individual receiving the treatment with thioproline, pharmaceutical salts and/or prodrugs thereof, is an individual, such as a healthy individual, who does not require losing weight due to a therapeutic indication, but who desires maintaining a body weight at normal values and maintaining or improving her/his bodily appearance from the essentially cosmetic point of view.

In a particular embodiment, to put said method into practice an enantiomeric mixture of thioproline, such as a racemic mixture or a mixture enriched with one of said d or 1 enantiomers of thioproline is used. In another particular embodiment, the *1* enantiomer of thioproline (*1*-thioproline) is used.

Thioproline, pharmaceutical salts and/or prodrugs thereof can be administered in the form of a pharmaceutical composition as has been previously mentioned and through any of the previously indicated administration routes, for example by oral, parenteral, rectal route, etc., preferably by oral route. In general, the effective amount of thioproline, pharmaceutical salts and/or prodrugs thereof, to be administered will depend on, among other factors, the individual to be treated, the chosen dosage form, etc. The doses mentioned in this invention must be considered only as a guideline for a person skilled in the art, and he must adjust the doses according to the previously mentioned variables. Nevertheless, as previously indicated, thioproline can be administered one or more times a day, for example 1 or 2 times a day, in a typical total daily amount comprised between 500 and 1000 mg/day.

Likewise, in this case the administration of thioproline, pharmaceutical salts and/or prodrugs thereof can also be done alone or in combination with additional drugs useful for reducing the body weight to provide a combined therapy, which additional drugs may or may not form part of the same pharmaceutical composition comprising thioproline, pharmaceutical salts and/or prodrugs thereof provided by this invention; in the event they do not, they will be administered simultaneously or sequentially in relation to the administration of the pharmaceutical composition comprising thioproline, pharmaceutical salts and/or prodrugs thereof provided by this invention. Illustrative, non-limiting examples of said additional drugs that can be used to provide combination therapy include anorexia-inducing agents, appetite suppressants, etc.

The following examples illustrate the invention and must not be considered as limiting of the scope of same.

### Example 1

### Assessment of the effect of thioproline on feeding behavior

### 1. Materials and Methods

### Animals

To study the effect of thioproline, particularly of 1-thioproline, on feeding behavior, CD-1 Swiss/UCádiz mice bred in the Animal Experimentation department of the Universidad de Cádiz were used.

The mice strain used in this study, CD-1 Swiss/UCádiz, is a type of mice with accelerated senescence similar to the AKR, SAM, NZB/Lac, and SJL/J mice having a mean life span of 36-57 weeks and a maximum life expectancy of 52-83 weeks. These mice have been used in the Universidad de Cádiz in several survival studies (Navarro A, Gomez C, Sanchez-Pino MJ, Gonzalez H, Bandez MJ, Boveris AD and Boveris A. Vitamin E at high doses improves survival, neurological performance, and brain mitochondrial function in aging male mice. Am J Physiol Regul Integr Comp Physiol 289: R1392-R1399, 2005; Navarro A, Gomez C, Lopez-Cepero JM and Boveris A. Beneficial effects of moderate exercise on mice aging: survival, behavior, oxidative stress, and mitochondrial electron transfer. Am J Physiol Regul Integr Comp Physiol 286: R505-R511, 2004; Navarro A, Sanchez Del Pino MJ, Gomez C, Peralta JL and Boveris A. Behavioral dysfunction, brain oxidative stress, and impaired mitochondrial electron transfer in aging mice. Am J Physiol Regul Integr Comp Physiol 282: R985-R992, 2002).

### Treatment

All the experiments were performed in accordance to the current law for animal experimentation (European Community Directive 86/609/EEC).

The mice were stabled in groups of 5 animals at 22 ± 2°C with 12 h/12 h of light/dark cycles and with free access, *ad libitum*, to food and water.

The control group received standard laboratory animal food (A04 diet, Panlab LS, Barcelona, Spain), whereas the 1-thioproline-supplemented mice received the same food as the mice in the control group, but supplemented with 2.0 g of 1-thioproline (Sigma Aldrich) / kg of food from 28 weeks of age and until the end of their lives.

The food was weighed daily. The mice were weighed weekly, and it was periodically controlled that the animals were pathogen-free.

### 2. Results

The obtained results are shown in the Figure 1, where significant differences between the body weight variation of the control group and the group treated with 1-thioproline can be observed.

As can be seen in said Figure 1, the body weight curves according to time show that, after 12 weeks of receiving an 1-thioproline-supplemented diet, the mice registered a smaller weight increase than the increase observed in the control group. The average weights at certain times for both groups of animals are shown in Table 1.

**Table 1**

| Average weight of the control and 1-thioproline-supplemented mice | | | | |
|---|---|---|---|---|
| | Weight (g) | | | |
| Age | 28 weeks | 52 weeks | 78 weeks | 100 weeks |
| Control mice | 43.6 ± 0.4 4 | 49.4 ± 0.5 5 | 54.9 ± 0.5* | 55 ± 0.5* |
| 1-thioproline-supplemented mice | 42.7 ± 0.4 4 | 47.2 ± 0.4 4 | 48.5 ± 0.5 | 49.4 ± 0.5 |
| Variation (%) | 2.06 | 4.45 | 11.65 | 10.18 |

| | | | | |
|---|---|---|---|---|
| The weight values in grams correspond to the average data ± SEM. * p < 0.01. | | | | |

The variation expresses the percentage reduction in weight between the supplemented mice and the mice of the control group in the indicated periods.

As can be seen, at the same ages, considering the time points of 52, 78, and 100 weeks of life, the *1*-thioproline-supplemented mice weighed (approximately) 4%, 11% and 10% less than the control animals.

The average daily intake *ad libitum* of the control mice was 6.3 ± 0.03 g/day, whereas the average daily intake *ad libitum* of the 1-thioproline-supplemented mice was 5.1 ± 0.03 g/day, which therefore involves a spontaneous reduction of the intake of approximately 20% in the 1-thioproline-supplemented mice.

In summary, the results obtained with this assay show that the mice with an 1-thioproline-supplemented diet reduced their body weight after 12 weeks of intake of the compound and that the mice supplemented with the standard diet (control animals) weighed more during their entire life, which evidences the efficacy of *1*-thioproline in body weight reduction.

### Example 2

### Assessment of the effect of thioproline on neurological capacities

The animals of Example 1 were subjected to two tests once a week to evaluate neurological capacities, specifically to the tightrope test and the T maze test.

### 1. Materials and Methods

### Animals

The assayed animals were the animals mentioned in Example 1, i.e. the group of control mice and the group of mice with an *1*-thioproline-supplemented diet according to the treatment described in the Example 1.

### Tightrope test

This test allows evaluating neuromuscular coordination. The mice were suspended in the center of a 60 cm wide tightrope and the time it took them to reach one of the columns located at the end of the rope was measured. The test was considered positive when the animal reached one of the ends in less than 60 seconds. T maze test

This test measures spontaneous cognitive neurological activity. The mice were placed in a T maze with 50 cm arms, and the test was considered successful when the mice reached the intersection in less than 60 seconds.

### 2. Results

The obtained results in the tightrope test are shown in Figure 2A and the results obtained in the T maze test are shown in Figure 2B. As can be seen, the mice with an *1*-thioproline-supplemented diet showed an improvement of 17-39% in the neurological tests compared to the mice of the control group (without supplement).

### EXAMPLE 3

### Assessment of the effect of thioproline on survival

The date on which each mouse died (Example 1) was controlled to be able to obtain the survival curves.

The obtained results are shown in Figure 3. The 1-thioproline-supplemented mice showed a prolongation of their mean life span of approximately 29% (they went from 62 weeks to 80 weeks) and a prolongation of approximately 23% of their maximum life (they went from 118 weeks to 145 weeks).

## Claims

1. A use of thioproline, pharmaceutically acceptable salts and/or prodrugs thereof in the preparation of a pharmaceutical composition for reducing body weight.

2. The use according to claim 1, wherein said pharmaceutical composition is intended for the treatment of overweight or obesity.

3. The use according to claim 1 or 2, wherein said pharmaceutical composition is a pharmaceutical composition intended to be administered by oral or parenteral route.

4. The use according to claim 3, wherein said pharmaceutical composition is administered by oral route.

5. The use according to any of claims 1 to 4, wherein said pharmaceutical composition comprises 1-thioproline or a mixture of thioproline enantiomers.

6. The use according to any of claims 1 to 5, wherein the pharmaceutical composition comprising thioproline is administered in combination with another additional drug useful for reducing body weight.

7. A method for improving the bodily appearance of an individual comprising administering thioproline, pharmaceutically acceptable salts and/or prodrugs thereof to said individual in an effective amount for reducing appetite and repeating said dose until achieving a cosmetically beneficial loss of body weight.

8. The method according to claim 7, in which thioproline, pharmaceutically acceptable salts and/or prodrugs thereof are administered by oral, parenteral or rectal route, preferably by oral route.

9. The method according to any of claims 7 or 8, comprising administering *1*-thioproline or a mixture of thioproline enantiomers.

10. The method according to any of claims 7 to 9, in which thioproline is administered in combination with another additional drug useful for reducing body weight.
